# EUROPEAN PATENT APPLICATION

(11) **EP 4 691 373 A1**
(43) Date of publication of application: **11.02.2026**
(21) Application number: 24779460.5
(22) Date of filing: 13.03.2024
(51) Int. Cl.: A61B 8/08, A61B 8/00

(54) **ULTRASONIC DIAGNOSTIC DEVICE AND CONTROL METHOD FOR ULTRASONIC DIAGNOSTIC DEVICE**

(30) Priority: 27.03.2023 JP 2023049517
(71) Applicant: FUJIFILM Corporation, Tokyo 106-8620 (JP)
(72) Inventor: KATO Noriji, Tokyo 106-8620 (JP)
(74) Representative: Klunker IP Patentanwälte PartG mbB
(86) International application number: PCT/JP2024/009769
(87) International publication number: WO 2024/203331

(57) **Abstract**

An ultrasound diagnostic apparatus includes an image acquisition unit (32) that performs a scan with an ultrasound probe (1) to continuously acquire ultrasound images of a plurality of frames in which a breast is imaged, a mammary gland region extraction unit (25) that extracts a mammary gland region from each of the ultrasound images of the plurality of frames, a low-brightness region extraction unit (26) that extracts a low-brightness region in which brightness is equal to or less than a predetermined brightness threshold value from the mammary gland region and that calculates an area of the low-brightness region, a region-of-interest extraction unit (27) that extracts a region of interest in which an area change rate is equal to or less than a predetermined change rate threshold value from a time-series change in the area of the low-brightness region in the plurality of frames, and an evaluation unit (28) that evaluates a risk of breast cancer based on a ratio of an area of the region of interest in the plurality of frames to an area of the mammary gland region in the plurality of frames.

## Description

### BACKGROUND OF THE INVENTION

### 1. Field of the Invention

The present invention relates to an ultrasound diagnostic apparatus used for an examination of a breast of a subject and a method of controlling the ultrasound diagnostic apparatus.

### 2. Description of the Related Art

In related art, in the medical field, an ultrasound diagnostic apparatus using ultrasound images is put into practical use. In general, the ultrasound diagnostic apparatus comprises an ultrasound probe provided with a transducer array and an apparatus body connected to the ultrasound probe, in which an ultrasound beam is transmitted from the ultrasound probe toward a subject, an ultrasound echo from the subject is received by the ultrasound probe, and a reception signal is electrically processed to generate the ultrasound image.

A composition of a fat tissue and a mammary gland tissue in a breast varies depending on a person, but an anatomical structure of the breast is common, and a primary lactiferous duct branches into extralobular ducts, which in turn connect to numerous lobules, in the mammary gland tissue. Stroma is present around the lobules, and mammary gland tissue is composed of the lobules together with the stroma.

It is known that two types of stroma exist around the lobules, that is, perilobular stroma and edematous stroma. The perilobular stroma exists along a structure from the lobule to the mammary duct, and includes many collagen fibers. Meanwhile, the edematous stroma fills the spaces between the perilobular stroma, is rich in extracellular matrix, with a mixture of collagen fibers and fat, and contains fewer collagen fibers as compared to the perilobular stroma.

In recent years, the concept of individualized risk management for patients has become widespread, but it is known that a ratio of the mammary gland region within the breast, especially a high-density mammary gland, is a risk factor for cancer. The ratio of the mammary gland region in the breast can be measured by using a mammography apparatus.

In Su Hyun Lee et al. "Glandular Tissue Component and Breast Cancer Risk in Mammographically Dense Breasts at Screening Breast US", Radiology, Volume 301, October 1, 2021, it is reported that a cancer is likely to occur in a case in which a ratio of a glandular tissue component (GTC) region including mammary ducts, lobules, and perilobular stroma in the mammary gland region is high even though the mammary gland regions are almost the same. That is, in addition to the ratio of the mammary gland region in the breast, a ratio of the GTC region in the mammary gland region may be a risk factor. This means a higher risk in a patient with less advanced atrophy of the lobule.

However, in the mammography apparatus, the perilobular stroma and the edematous stroma cannot be distinguished from each other, and the entire mammary gland tissue is observed as whitish, and as a result, the ratio of the GTC region in the mammary gland region cannot be measured.

JP2021-185970A detects an apparatus that extracts a suspected lesion region in a mammary gland region, which is a region suspected to have a lesion, from an ultrasound image.

### SUMMARY OF THE INVENTION

However, the ultrasound diagnostic apparatus of JP2021-185970A is intended to detect the suspected lesion region in the mammary gland region, and is not interested in evaluating the GTC region. Therefore, there is a problem in that the risk of breast cancer in the mammary gland region cannot be considered in detail.

In addition, since both the GTC region and the fat region are depicted as low-echo regions, that is, low-brightness regions in the ultrasound image, in a case in which the GTC region is manually evaluated as disclosed in Su Hyun Lee et al. "Glandular Tissue Component and Breast Cancer Risk in Mammographically Dense Breasts at Screening Breast US", Radiology, Volume 301, October 1, 2021, the user, such as a doctor, needs to determine the GTC region and the fat region, and thus it is difficult to evaluate the GTC region with high accuracy, and there is a case in which the user cannot consider the risk of breast cancer in the mammary gland region with high accuracy.

The present invention has been made in order to solve such a problem in the related art, and an object of the present invention is to provide an ultrasound diagnostic apparatus that enables a user to accurately consider a risk of breast cancer in a mammary gland region of a subject even in a case in which a fat region exists in the mammary gland region.

It is possible to achieve the above-described object with the following configurations.
[1] An ultrasound diagnostic apparatus comprising: an ultrasound probe; an image acquisition unit that performs a scan with the ultrasound probe to continuously acquire ultrasound images of a plurality of frames in which a breast of a subject is imaged; a mammary gland region extraction unit that extracts a mammary gland region from each of the ultrasound images of the plurality of frames; a low-brightness region extraction unit that extracts a low-brightness region in which brightness is equal to or less than a predetermined brightness threshold value from the mammary gland region extracted by the mammary gland region extraction unit and that calculates an area of the low-brightness region for each frame; a region-of-interest extraction unit that extracts a region of interest in which an area change rate is equal to or less than a predetermined change rate threshold value from a time-series change in the area of the low-brightness region in the plurality of frames; and an evaluation unit that evaluates a risk of breast cancer based on a ratio of an area of the region of interest in the plurality of frames extracted by the region-of-interest extraction unit to an area of the mammary gland region in the plurality of frames extracted by the mammary gland region extraction unit.
[2] The ultrasound diagnostic apparatus according to [1], in which the region-of-interest extraction unit selects a frame in which a frequency is equal to or less than a predetermined frequency threshold value by performing Fourier transform on time-series data of a total area of the low-brightness region in each of the plurality of frames, and extracts the low-brightness region in the selected frame as the region of interest.
[3] The ultrasound diagnostic apparatus according to [1], in which the region-of-interest extraction unit calculates an area change over a predetermined number of frames for each low-brightness region in the plurality of frames, and extracts, as the region of interest, the low-brightness region in which the calculated area change is equal to or less than a predetermined area change threshold value.
[4] The ultrasound diagnostic apparatus according to any one of [1] to [3], in which the low-brightness region extraction unit extracts a pixel at which brightness is equal to or less than the predetermined brightness threshold value from the mammary gland region extracted by the mammary gland region extraction unit, and calculates an area occupied by the extracted pixel as the area of the low-brightness region for each frame.
[5] The ultrasound diagnostic apparatus according to any one of [1] to [3], in which the low-brightness region extraction unit extracts the low-brightness region by binarizing the mammary gland region extracted by the mammary gland region extraction unit based on the predetermined brightness threshold value.
[6] The ultrasound diagnostic apparatus according to [1], further comprising: a low-brightness region integration unit that generates a continuous low-brightness region by integrating a plurality of the low-brightness regions that are extracted by the low-brightness region extraction unit in adjacent frames among the plurality of frames and that have overlapping portions, in which the region-of-interest extraction unit calculates a variance of the area of the low-brightness region in each frame in the continuous low-brightness region for each continuous low-brightness region, and extracts, as the region of interest, the low-brightness region in each frame in the continuous low-brightness region in which the calculated variance is equal to or less than a predetermined variance threshold value.
[7] The ultrasound diagnostic apparatus according to any one of [1] to [6], further comprising: a frame interval adjustment unit that calculates a position change amount of an imaging point in the ultrasound images of adjacent frames among the plurality of frames and that adjusts a time interval of the adjacent frames in accordance with the calculated position change amount, in which the region-of-interest extraction unit extracts the region of interest from the time-series change in the area of the low-brightness region in the plurality of frames based on the time interval adjusted by the frame interval adjustment unit.
[8] The ultrasound diagnostic apparatus according to [2], further comprising: a monitor that displays the ultrasound image; and a frame specifying unit that specifies a frame in which a frequency is greater than the predetermined frequency threshold value by performing inverse Fourier transform on the time-series data of the total area of the low-brightness region in each of the plurality of frames, the time-series data having been Fourier-transformed by the region-of-interest extraction unit, in which the ultrasound image of the frame specified by the frame specifying unit is displayed on the monitor.
[9] A method of controlling an ultrasound diagnostic apparatus, the method comprising: performing a scan with an ultrasound probe to continuously acquire ultrasound images of a plurality of frames in which a breast of a subject is imaged; extracting a mammary gland region from each of the ultrasound images of the plurality of frames; extracting a low-brightness region in which brightness is equal to or less than a predetermined brightness threshold value from the extracted mammary gland region and calculating an area of the low-brightness region for each frame; extracting a region of interest in which an area change rate is equal to or less than a predetermined change rate threshold value from a time-series change in the area of the low-brightness region in the plurality of frames; and evaluating a risk of breast cancer based on a ratio of an area of the extracted region of interest in the plurality of frames to an area of the extracted mammary gland region in the plurality of frames.

According to the aspects of the present invention, the ultrasound diagnostic apparatus comprises: the ultrasound probe; the image acquisition unit that performs the scan with the ultrasound probe to continuously acquire the ultrasound images of the plurality of frames in which the breast of the subject is imaged; the mammary gland region extraction unit that extracts the mammary gland region from each of the ultrasound images of the plurality of frames; the low-brightness region extraction unit that extracts the low-brightness region in which the brightness is equal to or less than the predetermined brightness threshold value from the mammary gland region extracted by the mammary gland region extraction unit and that calculates the area of the low-brightness region for each frame; the region-of-interest extraction unit that extracts the region of interest in which the area change rate is equal to or less than the predetermined change rate threshold value from the time-series change in the area of the low-brightness region in the plurality of frames; and the evaluation unit that evaluates the risk of breast cancer based on the ratio of the area of the region of interest in the plurality of frames extracted by the region-of-interest extraction unit to the area of the mammary gland region in the plurality of frames extracted by the mammary gland region extraction unit, so that the user can accurately consider the risk of breast cancer in the mammary gland region of the subject even in a case in which the fat region exists in the mammary gland region.

### BRIEF DESCRIPTION OF THE DRAWINGS

FIG. 1 is a block diagram showing a configuration of an ultrasound diagnostic apparatus according to Embodiment 1 of the present invention.
FIG. 2 is a block diagram showing an internal configuration of a transmission and reception circuit according to Embodiment 1.
FIG. 3 is a block diagram showing an internal configuration of an image generation unit according to Embodiment 1.
FIG. 4 is a diagram showing an ultrasound image obtained by imaging a mammary gland region of a subject.
FIG. 5 is a diagram showing an ultrasound image including a mammary gland region in which a GTC region is imaged.
FIG. 6 is an example of a graph showing a relationship between a frame number of the ultrasound image and an area of a low-brightness region.
FIG. 7 is an example of a graph obtained by performing Fourier transform on the relationship between the frame number of the ultrasound image and the area of the low-brightness region.
FIG. 8 is a flowchart showing an operation in Embodiment 1.
FIG. 9 is a block diagram showing a configuration of an ultrasound diagnostic apparatus according to Embodiment 2 of the present invention.
FIG. 10 is a block diagram showing a configuration of an ultrasound diagnostic apparatus according to Embodiment 3 of the present invention.
FIG. 11 is a block diagram showing a configuration of an ultrasound diagnostic apparatus according to Embodiment 4 of the present invention.

### DESCRIPTION OF THE PREFERRED EMBODIMENTS

Hereinafter, embodiments of the present invention will be described with reference to the accompanying drawings.

The following configuration requirements are described based on a representative embodiment of the present invention, but the present invention is not limited to such an embodiment.

In addition, the present specification, a numerical range represented by "to" means a range including numerical values described before and after "to", both ends inclusive, as a lower limit value and an upper limit value.

In the present specification, "same" and "identical" include an error range that is generally allowed in the technical field.

### [Embodiment 1]

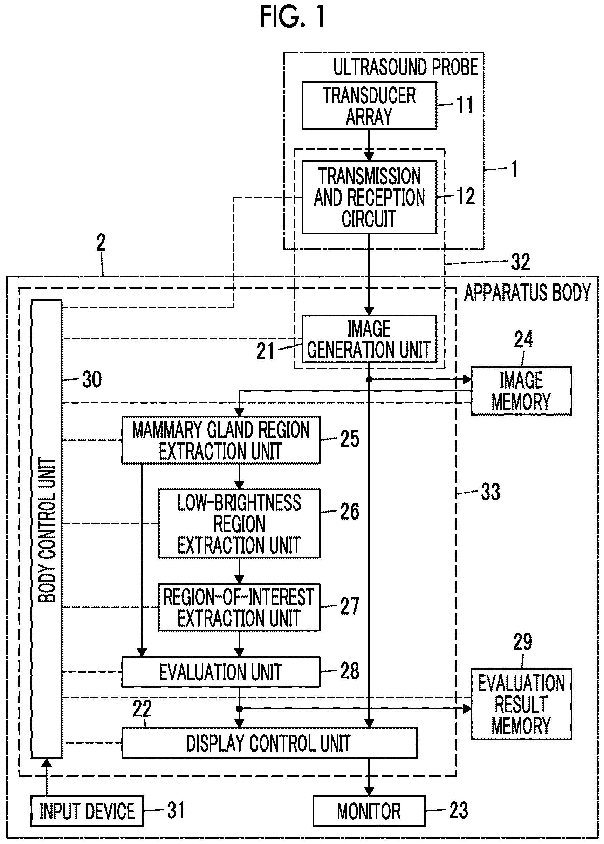

FIG. 1 shows a configuration of an ultrasound diagnostic apparatus according to the embodiment of the present invention. The ultrasound diagnostic apparatus comprises an ultrasound probe 1 and an apparatus body 2. The ultrasound probe 1 and the apparatus body 2 are wired-connected to each other through a cable (not shown).

The ultrasound probe 1 includes a transducer array 11 and a transmission and reception circuit 12 connected to the transducer array 11.

The apparatus body 2 includes an image generation unit 21 connected to the transmission and reception circuit 12 of the ultrasound probe 1, a display control unit 22 and a monitor 23 are connected sequentially to the image generation unit 21, and an image memory 24 is connected to the image generation unit 21. A mammary gland region extraction unit 25 is connected to the image memory 24. A low-brightness region extraction unit 26 and a region-of-interest extraction unit 27 are connected sequentially to the mammary gland region extraction unit 25. An evaluation unit 28 is connected to the mammary gland region extraction unit 25 and the region-of-interest extraction unit 27. An evaluation result memory 29 and the display control unit 22 are connected to the evaluation unit 28.

In addition, a body control unit 30 is connected to the transmission and reception circuit 12, the image generation unit 21, the display control unit 22, the image memory 24, the mammary gland region extraction unit 25, the low-brightness region extraction unit 26, the region-of-interest extraction unit 27, the evaluation unit 28, and the evaluation result memory 29. An input device 31 is connected to the body control unit 30. Further, the transmission and reception circuit 12 and the image generation unit 21 constitute an image acquisition unit 32. The image generation unit 21, the display control unit 22, the mammary gland region extraction unit 25, the low-brightness region extraction unit 26, the region-of-interest extraction unit 27, the evaluation unit 28, and the body control unit 30 constitute a processor 33 for the apparatus body 2.

The transducer array 11 of the ultrasound probe 1 includes a plurality of ultrasound transducers arranged in a one-dimensional or two-dimensional manner. Each of these transducers transmits an ultrasound wave in response to a drive signal supplied from the transmission and reception circuit 12, receives a reflected wave from a subject, and outputs an analog reception signal. Each transducer is formed by, for example, forming electrodes on both ends of a piezoelectric body consisting of a piezoelectric single crystal represented by lead zirconate titanate (PZT), a polymeric piezoelectric element represented by poly vinylidene di fluoride (PVDF), or a piezoelectric single crystal represented by a lead magnesium niobate-lead titanate (PMN-PT) solid solution.

An image acquisition unit 32 composed of the transmission and reception circuit 12 and the image generation unit 21 continuously performs a scan with the ultrasound probe 1 to acquire ultrasound images of a plurality of frames in which the breast of the subject is imaged.

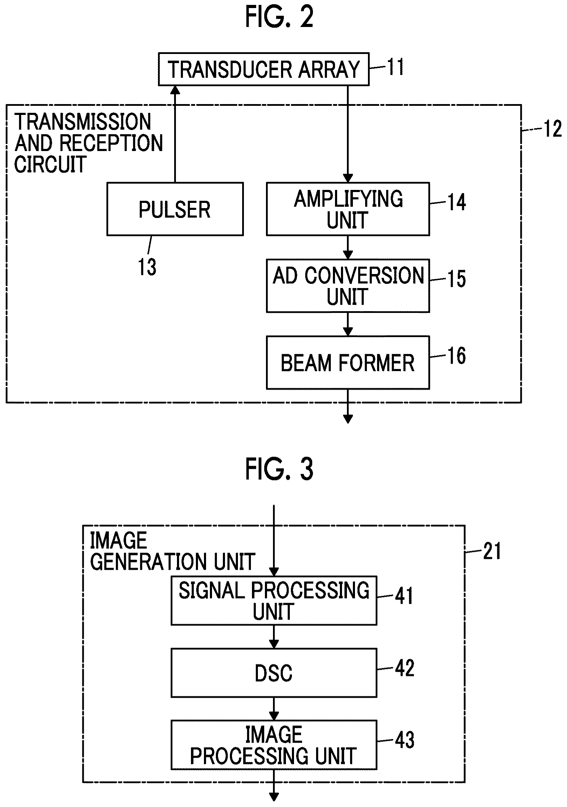

The transmission and reception circuit 12 transmits the ultrasound wave from the transducer array 11 and generates a sound ray signal based on the reception signal acquired by the transducer array 11, under the control of the body control unit 30. The transmission and reception circuit 12 includes, as shown in FIG. 2, a pulser 13 connected to the transducer array 11, and an amplifying unit 14, an analog-digital (AD) conversion unit 15, and a beam former 16 which are sequentially connected in series to the transducer array 11.

The pulser 13 includes, for example, a plurality of pulse generators, adjusts a delay amount of each drive signal based on a transmission delay pattern selected in accordance with a control signal from the body control unit 30 such that ultrasound waves to be transmitted from the plurality of transducers of the transducer array 11 form a ultrasound beam, and supplies the drive signal of which the delay amount has been adjusted, to the plurality of transducers. As described above, in a case in which a pulsed or continuous wave voltage is applied to the electrodes of the transducers of the transducer array 11, the piezoelectric body expands and contracts to generate a pulsed or continuous wave ultrasound wave from each transducer, and the ultrasound beam is formed from the combined wave of these ultrasound waves.

The transmitted ultrasound beam is reflected by a target, for example, a part of the subject, and an ultrasound echo propagates toward the transducer array 11 of the ultrasound probe 1. The ultrasound echo propagating toward the transducer array 11 in this manner is received by each of the transducers constituting the transducer array 11. In such a case, each transducer constituting the transducer array 11 expands and contracts by receiving the propagating ultrasound echo to generate the reception signal that is an electric signal, and outputs the reception signal to the amplifying unit 14.

The amplifying unit 14 amplifies the signal input from each of the transducers constituting the transducer array 11 and transmits the amplified signal to the AD conversion unit 15. The AD conversion unit 15 converts the signal transmitted from the amplifying unit 14 into digital reception data, and transmits the reception data to the beam former 16. The beam former 16 performs so-called reception focus processing by giving and adding delay with respect to each reception data converted by the AD conversion unit 15, in accordance with a sound velocity or a sound velocity distribution set based on a reception delay pattern selected according to a control signal from the body control unit 30. By the reception focus processing, a sound ray signal is acquired in which each piece of the reception data converted by the AD conversion unit 15 is phased and added and the focus of the ultrasound echo is narrowed.

The image generation unit 21 of the apparatus body 2 has, as shown in FIG. 3, a configuration in which a signal processing unit 41, a digital scan converter (DSC) 42, and an image processing unit 43 are sequentially connected in series.

The signal processing unit 41 performs, on the sound ray signal transmitted from the transmission and reception circuit 12 of the ultrasound probe 1, correction of attenuation caused by a distance in accordance with a depth of a reflection position of the ultrasound wave and then performs envelope detection processing, and thereby generates an ultrasound image signal (B-mode image signal), which is tomographic image information related to tissues in the subject.

The DSC 42 converts (raster-converts) the ultrasound image signal generated by the signal processing unit 41 into an image signal in accordance with a normal television signal scanning method.

The image processing unit 43 performs various types of necessary image processing, such as gradation processing, on the ultrasound image signal input from the DSC 42, and then outputs the signal representing the ultrasound image to the display control unit 22 and the image memory 24. The signal representing the ultrasound image generated by the image generation unit 21 in this way will be simply referred to as the ultrasound image. The image generation unit 21 can also output the ultrasound image signal before being processed by the DSC 42 or the ultrasound image signal immediately after being processed by the DSC 42 to the image memory 24. In this case, the image generation unit 21 can generate the ultrasound image by reading out these signals from the image memory 24 and performing processing using the DSC 42 or the image processing unit 43.

The image memory 24 is a memory that stores the ultrasound image generated by the image generation unit 21 under the control of the body control unit 30. For example, the image memory 24 can store a plurality of frames of ultrasound images generated by the image generation unit 21 in correspondence with diagnosis on a mammary gland region of a breast of the subject.

As the image memory 24, for example, a recording medium such as a flash memory, a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), or a universal serial bus memory (USB memory), can be used.

The mammary gland region extraction unit 25 detects a breast region of the subject from the ultrasound image read out from the image memory 24, and extracts the mammary gland region from the detected breast region.

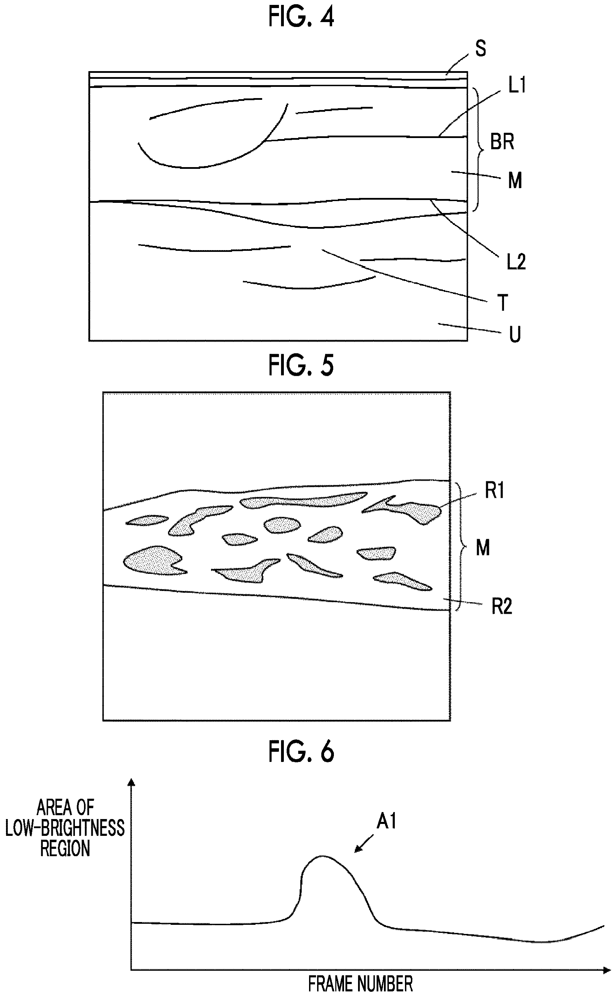

FIG. 4 shows an example of an ultrasound image U in which the breast of the subject is imaged. The ultrasound image U is a tomographic image captured by bringing a distal end of the ultrasound probe 1 into contact with the breast of the subject, in which a skin S of the subject is shown in an upper end of the ultrasound image U representing a shallowest portion, and a pectoralis major T is shown in a lower portion of the ultrasound image U representing a deeper portion. The mammary gland region extraction unit 25 can recognize a skin S and a pectoralis major T from the ultrasound image U and detect a deep region between the skin S and the pectoralis major T as a breast region BR.

As shown in FIG. 4, the mammary gland region extraction unit 25 can recognize a front boundary line L1 located on a shallower side and a rear boundary line L2 located on a deeper side in the detected breast region BR, and can extract a deep region between the front boundary line L1 and the rear boundary line L2 as a mammary gland region M.

In order to detect the breast region BR and to extract the mammary gland region M described above, the mammary gland region extraction unit 25 can perform image recognition using at least one of template matching, an image analysis technique using a feature value, such as adaptive boosting (AdaBoost), support vector machine (SVM), or scale-invariant feature transform (SIFT), or a determination model that has been trained by using a machine learning technique such as deep learning.

The determination model is a trained model that has learned the breast region BR and the mammary gland region M (segmentation) of the breast region BR in a training ultrasound image obtained by imaging the breast.

The low-brightness region extraction unit 26 has a predetermined brightness threshold value for the ultrasound image U, and, as shown in FIG. 5, extracts a low-brightness region R1 in which brightness is equal to or less than the brightness threshold value from the mammary gland region M extracted by the mammary gland region extraction unit 25, and calculates an area of the extracted low-brightness region R1 for each frame.

Here, the low-brightness region R1 includes a glandular tissue component (GTC) region and a fat region. The GTC region consists of mammary ducts, lobules, and perilobular stroma in the mammary gland region M, and edematous stroma R2 fills a space between the perilobular stroma. Since the edematous stroma R2 is rich in extracellular matrix and contains coexisting fat, in a case of observing the mammary gland region M using the ultrasound image U, the edematous stroma R2 has a high echo level (high-echo) and appears bright. On the other hand, the mammary ducts, the lobules, and the perilobular stroma constituting the GTC region have relatively low echo levels (low-echo), and have lower brightness than the edematous stroma R2. The fat region also has a low echo level and low brightness, as in the GTC region.

The low-brightness region extraction unit 26 can extract, for example, a pixel at which brightness is equal to or less than a predetermined brightness threshold value from the mammary gland region M extracted by the mammary gland region extraction unit 25, and calculate an area occupied by the extracted pixel as the area of the low-brightness region R1 for each frame.

In addition, the low-brightness region extraction unit 26 can also binarize the mammary gland region M extracted by the mammary gland region extraction unit 25 based on the predetermined brightness threshold value, and then use an algorithm such as a so-called watershed method, to extract the low-brightness region R1.

In addition, the low-brightness region extraction unit 26 can also extract the low-brightness region R1 by using a determination model that has been trained by using a machine learning technique such as deep learning. As the determination model, for example, a trained model, which has learned the low-brightness region R1 in the mammary gland region M in the training ultrasound image in which the breast is imaged, is used.

It should be noted that a predetermined constant value can be used as the brightness threshold value.

In addition, the low-brightness region extraction unit 26 may perform edge detection on the low-brightness region R1 in the ultrasound image U by image analysis, and automatically calculate the brightness threshold value based on a change in the brightness value in the detected edge portion, that is, a change in the brightness value of a plurality of pixels from the inside to the outside of the low-brightness region R1. In this way, the brightness threshold value suitable for the ultrasound image to be subjected to the image analysis can be automatically set.

Further, the low-brightness region extraction unit 26 can also set a value input by a user via the input device 31 as the brightness threshold value used for the binarization of the mammary gland region M. In this case, the low-brightness region extraction unit 26 can generate a binarized image of the mammary gland region M using an initial value of the brightness threshold value, and create a histogram of the brightness of the mammary gland region M in the ultrasound image U. In addition, the user can input an updated value of the brightness threshold value while referring to, for example, the binarized image generated using the initial value, the histogram of the brightness, and the ultrasound image U. In a case in which the brightness threshold value is input by the user in this way, the low-brightness region extraction unit 26 can update the binarized image using the brightness threshold value input by the user.

In a case in which the ultrasound images U of the plurality of frames are generated by the image generation unit 21 in a state in which the ultrasound probe 1 is moved on the breast of the subject by the user such as a doctor, the region-of-interest extraction unit 27 has a predetermined change rate threshold value for a time-series change in the area of the low-brightness region R1 in the ultrasound images U of the plurality of frames, and extracts a region of interest in which the area change rate is equal to or less than the change rate threshold value from the time-series change in the area of the low-brightness region R1 in the ultrasound images U of the plurality of frames. The area change rate of the low-brightness region R1 is represented by, for example, the area change amount of the low-brightness region R1 between the frames.

For example, as shown in FIG. 6, the region-of-interest extraction unit 27 creates time-series data of a total area of the low-brightness region R1 in each of the ultrasound images U of the plurality of frames by arranging the total area of the low-brightness region R1 in each of the ultrasound images U of the plurality of frames in the order of the frame numbers. In a case in which the fat region is present in the mammary gland region M, the fat region is shown in several frames that are consecutive in time series among the ultrasound images U of the plurality of frames acquired while the ultrasound probe 1 is moved, and thus the total area of the low-brightness region R1 in the ultrasound images U of several frames increases. As a result, in the time-series data, a portion A1 is generated in which the total area of the low-brightness region R1 is convex upward.

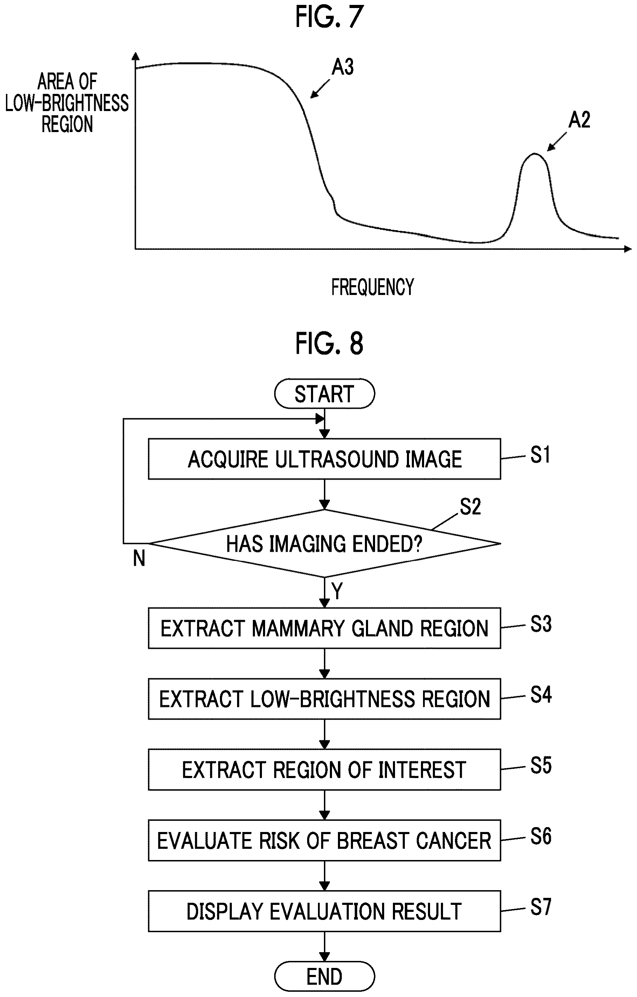

The region-of-interest extraction unit 27 can obtain Fourier transform data representing a relationship between the total area and a frequency of the low-brightness region R1 in each of the ultrasound images of the plurality of frames as shown in FIG. 7 by performing so-called Fourier transform on the time-series data. In a case in which the fat region is present in the mammary gland region M, in the time-series data, a upwardly convex portion A2 representing the presence of the fat region is generated, in a relatively high frequency band, corresponding to the portion A1 in which the total area of the low-brightness region R1 is convex upward in the Fourier transform data. Further, a portion A3 representing the presence of the GTC region is generated in a relatively low frequency band.

The region-of-interest extraction unit 27 can have a predetermined frequency threshold value as a rate change threshold value with respect to the area of the low-brightness region R1, exclude the frame including the fat region by selecting the frame in which a frequency is equal to or less than the frequency threshold value in the Fourier transform data, and extract the low-brightness region R1 in the selected frame as the region of interest. The region of interest extracted in this manner is a region in which the fat region is effectively excluded.

In addition, the region-of-interest extraction unit 27 can also obtain the region of interest in which the fat region is effectively excluded without using the Fourier transform. For example, the region-of-interest extraction unit 27 can have a predetermined area change threshold value for an area change in the low-brightness region R1 in the time-series data, calculate an area change over a predetermined number of frames for each low-brightness region R1 in the ultrasound images U of the plurality of frames based on the time-series data, and extract the low-brightness region R1 in which the calculated area change is equal to or less than the area change threshold value as the region of interest.

As a result, the upwardly convex portion A2 representing the presence of the fat region can be excluded from the time-series data as shown in FIG. 6, and thus the region of interest can be extracted as a region in which the fat region is effectively excluded.

The evaluation unit 28 evaluates a risk of breast cancer based on the ratio of the area of the region of interest in the ultrasound images U of the plurality of frames extracted by the region-of-interest extraction unit 27 to the area of the mammary gland region M in the ultrasound images U of the plurality of frames extracted by the mammary gland region extraction unit 25.

Here, since the region of interest is obtained by excluding the fat region from the low-brightness region R1 including the GTC region and the fat region, the ratio of the area of the region of interest calculated in this way means a ratio of the area of the GTC region to the area of the mammary gland region M.

It is generally known that the lobule atrophies with age, but there are research results that a risk of breast cancer is high in patients in whom the lobule does not atrophy, as disclosed in, for example, "Su Hyun Lee et al. "Glandular Tissue Component and Breast Cancer Risk in Mammographically Dense Breasts at Screening Breast US", Radiology, Volume 301, October 1, 2021".

The ratio of the area of the region of interest to the area of the mammary gland region M represents a degree of progression of the atrophy of the lobule, and can be used as a determination material for the risk of breast cancer. Therefore, the evaluation unit 28 can use, for example, an average value, a median value, or a sum value of the ratio of the area of the region of interest to the area of the mammary gland region M in the plurality of frames, as an evaluation result of the risk of breast cancer. The user, such as the doctor, can determine that, the lower the ratio of the area of the region of interest to the area of the mammary gland region M, the higher the risk of breast cancer.

In addition, for example, the evaluation unit 28 can store a predetermined breast cancer risk function for calculating a higher breast cancer risk value as the ratio of the area of the region of interest to the area of the mammary gland region M is lower, and use the calculated ratio of the area of the region of interest and the breast cancer risk value calculated based on the breast cancer risk function as the evaluation results of the risk of breast cancer. The user can determine that the higher the breast cancer risk value, the higher the risk of breast cancer.

In addition, the evaluation unit 28 can determine a category of the region of interest based on the ratio of the area of the region of interest to the area of the mammary gland region M, and use the category as the evaluation result of the risk of breast cancer.

The evaluation unit 28 can output, as the evaluation result, any one of a plurality of predetermined categories, for example, any one of two categories of Low and High as the category of the region of interest. Low indicates that the lobule atrophy has not progressed as much as in High. Further, the evaluation unit 28 can also output, for example, any one of four categories of Minimal, Mild, Moderate, and Marked as the category of the region of interest. Mild indicates that the atrophy of the lobule is not more advanced than that in Minimal, Moderate indicates that the atrophy of the lobule is not more advanced than that in Mild, and Marked indicates that the atrophy of the lobule is not more advanced than that in Moderate.

It is known that, in general, both the GTC region and the fat region are depicted as the low-brightness region R1 in the ultrasound image U. Therefore, since it is difficult for the user, such as the doctor, to accurately distinguish between the GTC region and the fat region, there is a case in which the user cannot accurately consider the risk of breast cancer in the mammary gland region M. In the ultrasound diagnostic apparatus according to the embodiment of the present invention, the region-of-interest extraction unit 27 extracts the region of interest in which the fat region is excluded from the low-brightness region R1 extracted by the low-brightness region extraction unit 26, and the evaluation unit 28 evaluates the risk of breast cancer using the region of interest, so that a highly accurate evaluation result can be obtained.

The evaluation result memory 29 stores the evaluation result of the risk of breast cancer by the evaluation unit 28. The user can, for example, read out the evaluation result via the input device 31 after the examination of the subject, and consider the risk of breast cancer in the breast of the subject based on the evaluation result. As the evaluation result memory 29, for example, recording media such as a flash memory, an HDD, an SSD, an FD, an MO disc, an MT, a RAM, a CD, a DVD, an SD card, and an USB memory can be used.

The display control unit 22 performs predetermined processing on the ultrasound image U sent from the image generation unit 21, the evaluation result of the risk of breast cancer by the evaluation unit 28, and the like under the control of the body control unit 30, and displays the ultrasound image U, the evaluation result of the risk of breast cancer, and the like on the monitor 23.

The monitor 23 displays the ultrasound image U and the like under the control of the display control unit 22, and includes, for example, a display device such as a liquid crystal display (LCD) or an organic electroluminescence display (organic EL display).

The body control unit 30 controls each unit of the apparatus body 2 and the transmission and reception circuit 12 of the ultrasound probe 1 based on a control program or the like, which is stored in advance.

The input device 31 is an input device used by the user to perform an input operation, and is configured by, for example, a device such as a keyboard, a mouse, a trackball, a touchpad, and a touch sensor disposed in a state of being superimposed on the monitor 23.

The processor 33 including the image generation unit 21, the display control unit 22, the mammary gland region extraction unit 25, the low-brightness region extraction unit 26, the region-of-interest extraction unit 27, the evaluation unit 28, and the body control unit 30 is configured by a central processing unit (CPU) and a control program for causing the CPU to execute various kinds of processing, but the processor 33 may be configured by using a field programmable gate array (FPGA), a digital signal processor (DSP), an application specific integrated circuit (ASIC), a graphics processing unit (GPU), or other integrated circuits (IC) or may be configured by a combination thereof.

In addition, the image generation unit 21, the display control unit 22, the mammary gland region extraction unit 25, the low-brightness region extraction unit 26, the region-of-interest extraction section 27, the evaluation unit 28, and the body control unit 30 of the processor 33 can also be configured by being integrated partially or entirely into one CPU or the like.

Hereinafter, an operation of the ultrasound diagnostic apparatus according to the embodiment will be described with reference to a flowchart shown in FIG. 8.

First, in step S1, the breast of the subject is imaged by using the ultrasound probe 1, and the ultrasound image U is acquired. In this case, under the control of the body control unit 30, the transmission and reception of the ultrasound waves from the plurality of transducers of the transducer array 11 are started in accordance with the drive signal from the pulser 13 of the transmission and reception circuit 12 of the ultrasound probe 1, the ultrasound echo from the inside of the breast of the subject is received by the plurality of transducers of the transducer array 11, the reception signal which is an analog signal is output to the amplifying unit 14 and is amplified by the amplifying unit 14, and the amplified reception signal is AD-converted by the AD conversion unit 15 to acquire the reception data.

The reception focus processing is performed on the reception data by the beam former 16, and the sound ray signal generated by the reception focus processing is transmitted to the image generation unit 21 of the apparatus body 2, and as a result, the ultrasound image U representing the tomographic image information of the breast of the subject is generated by the image generation unit 21. In this case, the signal processing unit 41 of the image generation unit 21 performs the correction of the attenuation in accordance with the depth of the reflection position of the ultrasound wave and the envelope detection processing on the sound ray signal, the DSC 42 performs the conversion into the image signal in accordance with the normal television signal scanning method, and the image processing unit 43 performs various types of necessary image processing such as gradation processing. The ultrasound image U acquired as described above is stored in the image memory 24.

In step S2, the body control unit 30 determines whether or not to end the capture of the ultrasound image U. The body control unit 30 can determine to end the capture of the ultrasound image U, for example, in a case in which an instruction to end the capture of the ultrasound image U is input by the user via the input device 31. In addition, the body control unit 30 can determine to continue the capture of the ultrasound image U, for example, in a case in which the instruction to end the capture of the ultrasound image U is not particularly input by the user via the input device 31.

In a case in which it is determined to continue the capture of the ultrasound image U in step S2, the processing returns to step S1, and a new ultrasound image U is acquired. In this way, as long as it is determined in step S2 to continue the capture of the ultrasound image U, the processing of step S1 and step S2 is repeated, and the ultrasound images U of the plurality of frames are acquired. In this case, the ultrasound images U of the plurality of frames are acquired by the user in a state in which the ultrasound probe 1 is moved on the breast of the subject.

In a case in which it is determined in step S2 to end the capture of the ultrasound image U, the processing proceeds to step S3.

In step S3, the mammary gland region extraction unit 25 detects the breast region BR of the subject from each of the ultrasound images U of the plurality of frames acquired by repeating step S1 and step S2, to extract the mammary gland region M from the detected breast region BR. The mammary gland region extraction unit 25 can perform the image recognition using at least one of template matching, an image analysis technique using a feature value, such as AdaBoost, SVM, or SIFT, or a determination model that has been trained using a machine learning technique such as deep learning, in order to detect the breast region BR and to detect the mammary gland region M, for example.

In step S4, for example, as shown in FIG. 5, the low-brightness region extraction unit 26 extracts the low-brightness region R1 in which the brightness is equal to or less than the predetermined brightness threshold value from the mammary gland region M extracted in step S2, and calculates the area of the low-brightness region R1 for each frame.

The low-brightness region extraction unit 26 can extract, for example, a pixel at which brightness is equal to or less than a predetermined brightness threshold value from the mammary gland region M extracted by the mammary gland region extraction unit 25, and calculate an area occupied by the extracted pixel as the area of the low-brightness region R1 for each frame. The low-brightness region extraction unit 26 can also extract the low-brightness region R1 by binarizing the mammary gland region M extracted by the mammary gland region extraction unit 25 based on the predetermined brightness threshold value.

In step S5, the region-of-interest extraction unit 27 extracts the region of interest in which the area change rate is equal to or less than the predetermined change rate threshold value from the time-series change of the area of the low-brightness region R1 in the plurality of frames extracted in step S4.

For example, the region-of-interest extraction unit 27 can acquire the Fourier transform data as shown in FIG. 6 by performing the Fourier transform on the time-series data of the total area of the low-brightness region R1 in each of the plurality of frames as shown in FIG. 5, select the frame in which the frequency is less than or equal to the frequency threshold value determined in the Fourier transform data, and extract the low-brightness region R1 in the selected frame as the region of interest. The region of interest extracted in this manner is a region in which the fat region is effectively excluded.

In addition, the region-of-interest extraction unit 27 can also calculate the area change over a predetermined number of frames for each low-brightness region R1 in the plurality of frames based on, for example, the time-series data as shown in FIG. 5, and extract the low-brightness region R1 in which the calculated area change is equal to or less than the predetermined area change threshold value as the region of interest. The region of interest extracted in this manner is also a region in which the fat region is effectively excluded.

In step S6, the evaluation unit 28 evaluates the risk of breast cancer based on the ratio of the area of the region of interest in the ultrasound images U of the plurality of frames extracted by the region-of-interest extraction unit 27 to the area of the mammary gland region M in the ultrasound images U of the plurality of frames extracted in step S5.

The evaluation unit 28 can use, for example, the average value, the median value, or the sum value of the ratio of the area of the region of interest to the area of the mammary gland region M in the plurality of frames, as the evaluation result of the risk of breast cancer. The user can determine that, the lower the ratio of the area of the region of interest to the area of the mammary gland region M, the higher the risk of breast cancer.

In addition, the evaluation unit 28 can also use, for example, the calculated ratio of the area of the region of interest and the breast cancer risk value calculated based on the predetermined breast cancer risk function for calculating a higher breast cancer risk value as the ratio of the area of the region of interest to the area of the mammary gland region M is lower, as the evaluation results of the risk of breast cancer. The user can determine that the higher the breast cancer risk value, the higher the risk of breast cancer.

The evaluation unit 28 can determine the category of the region of interest based on the ratio of the area of the region of interest to the area of the mammary gland region M, and use the category as the evaluation result of the risk of breast cancer. The evaluation unit 28 can output, as the evaluation result, any one of a plurality of predetermined categories, for example, any one of two categories of Low and High as the category of the region of interest. Further, the evaluation unit 28 can also output, for example, any one of four categories of Minimal, Mild, Moderate, and Marked as the category of the region of interest.

Although i is known that, in general, both the GTC region and the fat region are depicted as the low-brightness region R1 in the ultrasound image U, in step S6, the evaluation of the risk of breast cancer is performed using the region of interest in which the fat region is excluded from the low-brightness region R1 in the mammary gland region M, so that a highly accurate evaluation result can be obtained.

Finally, in step S7, the display control unit 22 displays the evaluation result of the risk of breast cancer obtained in step S7 on the monitor 23. The user can accurately consider the risk of breast cancer in the breast of the subject by referring to the evaluation result of the risk of breast cancer displayed on the monitor 23.

In a case in which the processing of step S7 is completed in this manner, the operation of the ultrasound diagnostic apparatus according to the flowchart of FIG. 8 is completed.

As described above, with the ultrasound diagnostic apparatus according to Embodiment 1, the mammary gland region extraction unit 25 extracts the mammary gland region M from each of the ultrasound images U of the plurality of frames, the low-brightness region extraction unit 26 extracts the low-brightness region R1 in which the brightness is equal to or less than the predetermined brightness threshold value from the mammary gland region M and calculates the area of the low-brightness region R1 for each frame, the region-of-interest extraction unit 27 extracts the region of interest in which the area change rate is equal to or less than the predetermined change rate threshold value from the time-series change of the area of the low-brightness region R1 in the plurality of frames, and the evaluation unit 28 evaluates the risk of breast cancer based on the ratio of the area of the region of interest in the plurality of frames to the area of the mammary gland region M in the plurality of frames, so that the user can accurately consider the risk of breast cancer in the mammary gland region M of the subject even in a case in which the fat region exists in the mammary gland region M.

A case has been described in which the transmission and reception circuit 12 is provided in the ultrasound probe 1, but the transmission and reception circuit 12 may be provided in the apparatus body 2.

A case has been described in which the image generation unit 21 is provided in the apparatus body 2, but the image generation unit 21 may be provided in the ultrasound probe 1.

The apparatus body 2 may be a so-called stationary type, a portable type that is easy to carry, or a so-called handheld type that is configured by, for example, a smartphone or a tablet type computer. As described above, the type of the device constituting the apparatus body 2 is not particularly limited.

A case has been described in which the ultrasound probe 1 and the apparatus body 2 are connected to each other in a wired manner, but the ultrasound probe 1 and the apparatus body 2 may be connected to each other in a wireless manner.

In addition, in the flowchart of FIG. 8, after the ultrasound images U of the plurality of frames are acquired by repeating step S1 and step S2, the processing of extracting the mammary gland region M in step S3 and the processing of extracting the low-brightness region R1 in step S4 are performed, but the processing of step S3 and the processing of step S4 may be performed each time the ultrasound image U is acquired in step S1, and then the determination processing of step S2 may be performed. In this case, it is possible to store the mammary gland region M extracted in step S3 and the low-brightness region R1 extracted in step S4 in the image memory 24 in association with the ultrasound image U acquired in step S1. In addition, in a case in which it is determined in step S2 to continue the capture of the ultrasound image U, the processing returns to step S1, and then, the processing of step S2 and the processing of step S3 are sequentially performed.

In addition, it has been described that, in step S2, the body control unit 30 determines whether or not to end the capture of the ultrasound image U based on the instruction of the user via the input device 31, but the method of determining whether or not to end the capture of the ultrasound image U is not particularly limited to this.

For example, the body control unit 30 can determine to end the capture of the ultrasound image U in a case in which the ultrasound image U of the predetermined frame is acquired, and can determine to continue the capture of the ultrasound image U in a case in which the number of frames of the acquired ultrasound image U is less than the predetermined number of frames. In addition, for example, the body control unit 30 can determine to end the capture of the ultrasound image U in a case in which a predetermined time has elapsed from the start of the capture of the ultrasound image U, and determine to continue the capture of the ultrasound image U in a case in which the time elapsed from the start of the capture of the ultrasound image U is not the predetermined time. In this way, the body control unit 30 automatically determines the end of the capture of the ultrasound image U, so that the user can save time and effort for inputting the instruction via the input device 31 and can smoothly perform the examination.

Further, the mammary gland region extraction unit 25 can determine whether or not the shape of the mammary gland region M extracted from the ultrasound images U of the plurality of frames is continuously changed in the ultrasound images U of the plurality of frames. In this case, for example, the mammary gland region extraction unit 25 can calculate similarity between the mammary gland regions M in the ultrasound images U of the frames adjacent in time series, determine that the shape of the mammary gland region M is continuously changed in a case in which the calculated similarity is equal to or less than a predetermined similarity threshold value, and determine that the shape of the mammary gland region M is not continuously changed in a case in which the calculated similarity is greater than the predetermined similarity threshold value.

In a case in which it is determined whether or not the shape of the mammary gland region M is continuous in the ultrasound images U of the plurality of frames, the body control unit 30 can end the subsequent processing or guide the user to acquire the ultrasound images U of the plurality of frames again, for example, by displaying a message on the monitor 23 in a case in which it is determined by the mammary gland region extraction unit 25 that the shape of the mammary gland region M is not continuously changed. As a result, it is possible to reliably acquire the ultrasound images U of the plurality of frames in which the shape of the mammary gland region M is continuously changed, and to improve the accuracy of the extraction of the region of interest in the region-of-interest extraction unit 27 and the evaluation of the risk of breast cancer by the evaluation unit 28.

### [Embodiment 2]

A continuous low-brightness region can be generated by integrating the low-brightness regions R1 that are adjacent in time series and have overlapping portions in the ultrasound images U of the plurality of frames, and the region of interest can be extracted based on the continuous low-brightness region.

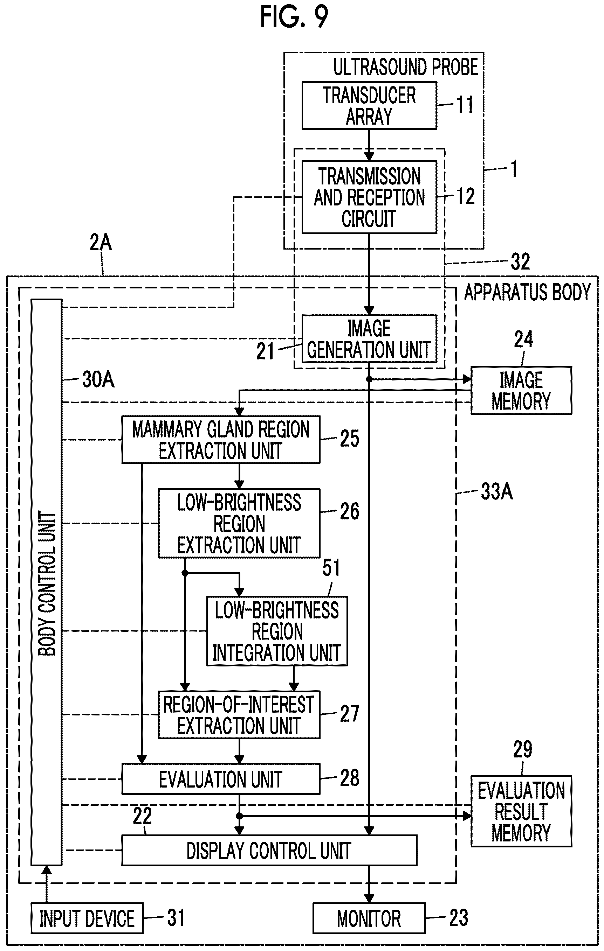

FIG. 9 shows a configuration of an ultrasound diagnostic apparatus according to Embodiment 2. The ultrasound diagnostic apparatus according to Embodiment 2 comprises an apparatus body 2A instead of the apparatus body 2 in the ultrasound diagnostic apparatus according to Embodiment 1 shown in FIG. 1. The apparatus body 2A further comprises a low-brightness region integration unit 51 and comprises a body control unit 30A instead of the body control unit 30, in the apparatus body 2 according to Embodiment 1.

In the apparatus body 2A, the low-brightness region integration unit 51 is connected to the low-brightness region extraction unit 26. In addition, the region-of-interest extraction unit 27 is connected to the low-brightness region extraction unit 26 and the low-brightness region integration unit 51. In addition, the low-brightness region integration unit 51 is connected to the body control unit 30A. In addition, the image generation unit 21, the display control unit 22, the mammary gland region extraction unit 25, the low-brightness region extraction unit 26, the region-of-interest extraction unit 27, the evaluation unit 28, the body control unit 30A, and the low-brightness region integration unit 51 constitute a processor 33A for the apparatus body 2A.

The low-brightness region integration unit 51 generates the continuous low-brightness region by integrating (associating) a plurality of low-brightness regions R1 extracted by the low-brightness region extraction unit 26 in the ultrasound images U of the frames adjacent in time series among the ultrasound images U of the plurality of frames generated by the image generation unit 21 and having overlapping portions in the ultrasound images U. The continuous low-brightness region generated in this way corresponds to the plurality of low-brightness regions R1. Therefore, it is possible to determine whether or not the fat region and the like other than the GTC region are included for each of the plurality of low-brightness regions R1, by a method using a variance of the area of the low-brightness region R1 described below.

The region-of-interest extraction unit 27 has a predetermined variance threshold value for the variance of the area of the low-brightness region R1 in the continuous low-brightness region, calculates, for each continuous low-brightness region, the variance of the area of the low-brightness region R1 in the ultrasound image U of each frame in the continuous low-brightness region, and extracts the low-brightness region R1 in each frame in the continuous low-brightness region in which the calculated variance is equal to or less than the variance threshold value as the region of interest.

Here, the continuous low-brightness region in which the variance of the area of the low-brightness region R1 is greater than a certain value is likely to include the fat region or the like different from the GTC region. Therefore, by extracting the low-brightness region R1 in each frame in the continuous low-brightness region in which the variance is equal to or less than the variance threshold value as the region of interest, the region of interest excluding the fat region and the like can be obtained.

The evaluation unit 28 evaluates the risk of breast cancer using the region of interest extracted by the region-of-interest extraction unit 27 as described above.

As described above, with the ultrasound diagnostic apparatus according to Embodiment 2, the low-brightness region integration unit 51 generates the continuous low-brightness region by integrating the plurality of low-brightness regions R1 extracted from frames adjacent to each other in time series among the ultrasound images U of the plurality of frames generated by the image generation unit 21 and having the overlapping portions, and the region-of-interest extraction unit 27 calculates, for the continuous low-brightness region, the variance of the area of the low-brightness region R1 in each frame in the continuous low-brightness region and extracts the low-brightness region R1 in each frame in the continuous low-brightness region in which the calculated variance is equal to or less than the variance threshold value as the region of interest, so that the region of interest in which the shape is continuously changed in the plurality of frames and the fat region is effectively excluded is obtained, and the accuracy of the evaluation of the risk of breast cancer can be improved.

### [Embodiment 3]

In Embodiments 1 and 2, since the user moves the ultrasound probe 1, a frame interval of the ultrasound images U of the plurality of frames that are consecutive in time series may not be constant. Therefore, the ultrasound diagnostic apparatus can also adjust the frame interval of the ultrasound images U of the plurality of frames.

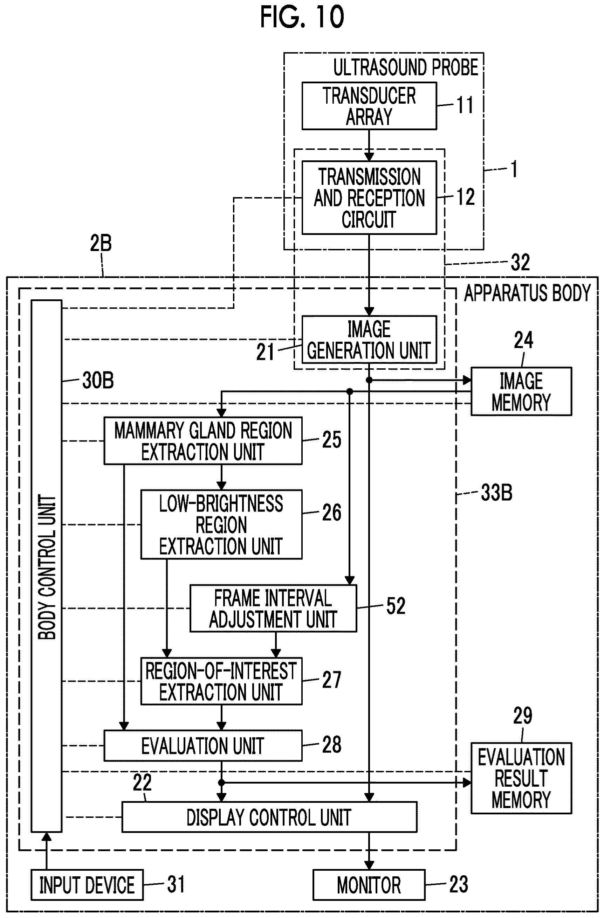

FIG. 10 shows a configuration of an ultrasound diagnostic apparatus according to Embodiment 3. The ultrasound diagnostic apparatus according to Embodiment 3 comprises an apparatus body 2B instead of the apparatus body 2 with respect to the ultrasound diagnostic apparatus according to Embodiment 1 shown in FIG. 1. The apparatus body 2B further comprises a frame interval adjustment unit 52 and comprises a body control unit 30B instead of the body control unit 30, in the apparatus body 2 according to Embodiment 1.

In the apparatus body 2B, the frame interval adjustment unit 52 is connected to the image memory 24. The frame interval adjustment unit 52 is connected to the region-of-interest extraction unit 27 and the body control unit 30B. In addition, the image generation unit 21, the display control unit 22, the mammary gland region extraction unit 25, the low-brightness region extraction unit 26, the region-of-interest extraction unit 27, the evaluation unit 28, the body control unit 30B, and the frame interval adjustment unit 52 constitute a processor 33B for the apparatus body 2B.

The frame interval adjustment unit 52 calculates a position change amount of an imaging point of the ultrasound image U in frames adjacent in time series among the ultrasound images U of the plurality of frames generated by the image generation unit 21, and adjusts a time interval between the adjacent frames in accordance with the calculated position change amount.

The frame interval adjustment unit 52 can calculate an image difference of the ultrasound images U of the frames adjacent in time series as the position change amount of the imaging point of the ultrasound image U, and adjust the time interval of the adjacent frames in accordance with the image difference. For example, the frame interval adjustment unit 52 can determine that a distance between the imaging points of the ultrasound images U of the adjacent frames is longer as the image difference is larger, to widen the time interval of the adjacent frames, and can determine that the distance between the imaging points of the ultrasound images U of the adjacent frames is shorter as the image difference is smaller, to narrow the time interval of the adjacent frames.

In this way, the frame interval adjustment unit 52 adjusts the frame interval of the ultrasound images U of the frames adjacent in time series to correspond to the distance between the imaging points of the ultrasound images U adjacent to each other.

The region-of-interest extraction unit 27 extracts the region of interest from the time-series change in the area of the low-brightness region R1 in the ultrasound images U of the plurality of frames based on the time interval adjusted by the frame interval adjustment unit 52. In this case, the region-of-interest extraction unit 27 can create corrected time-series data by correcting, for example, the time-series data of the area of the low-brightness region R1 as shown in FIG. 5 based on the time interval adjusted by the frame interval adjustment unit 52. The region-of-interest extraction unit 27 can extract the region of interest in which the fat region is excluded, by a method of performing Fourier transform on the corrected time-series data, a method of calculating the area change in the low-brightness region R1 based on the corrected time-series data, or the like.

The evaluation unit 28 evaluates the risk of breast cancer using the region of interest extracted by the region-of-interest extraction unit 27 as described above.

As described above, with the ultrasound diagnostic apparatus according to Embodiment 3, the frame interval adjustment unit 52 calculates the position change amount of the imaging point of the ultrasound image U in the frames adjacent in time series and adjusts the time interval between the frames adjacent in time series in accordance with the calculated position change amount, and the region-of-interest extraction unit 27 extracts the region of interest from the time-series change in the area of the low-brightness region R1 in the plurality of frames based on the adjusted time interval, so that, even in a case in which the movement speed of the ultrasound probe 1 by the user is not constant, it is possible to create the time-series data indicating the correct time-series change in the area of the low-brightness region R1 in the plurality of frames, and to extract the region of interest in which the fat region is reliably excluded. As a result, it is possible to improve the accuracy of the evaluation of the risk of breast cancer in the evaluation unit 28.

In a case in which the ultrasound diagnostic apparatus comprises a position sensor (not shown) that detects the position of the ultrasound probe 1, the frame interval adjustment unit 52 can calculate the position change amount of the imaging point of the ultrasound image U based on the position of the ultrasound probe 1 detected by the position sensor, and adjust the time interval between the frames adjacent in time series based on the calculated position change amount. As the position sensor, a magnetic sensor, an acceleration sensor, a gyro sensor, a distance measurement device such as a so-called light detection and ranging (LiDAR), a global positioning system (GPS) sensor, and the like can be used.

In addition, in a case in which the ultrasound diagnostic apparatus comprises an optical camera (not shown) that captures an optical image of the ultrasound probe 1, the frame interval adjustment unit 52 can calculate the position change amount of the imaging point of the ultrasound image U by analyzing the optical image of the ultrasound probe 1 captured by the optical camera, and adjust the time interval between the frames adjacent in time series based on the calculated position change amount.

In addition, the ultrasound diagnostic apparatus according to Embodiment 3 is configured by providing the apparatus body 2 with the frame interval adjustment unit 52 in the ultrasound diagnostic apparatus according to Embodiment 1, but may be configured by providing the apparatus body 2A with the frame interval adjustment unit 52 in the ultrasound diagnostic apparatus according to Embodiment 2.

### [Embodiment 4]

In a case in which the region-of-interest extraction unit 27 extracts the region of interest using the Fourier transform method, the region-of-interest extraction unit 27 can specify a frame in which a frequency is greater than a predetermined frequency threshold value based on the Fourier transform data, and display the ultrasound image U of the specified frame on the monitor 23.

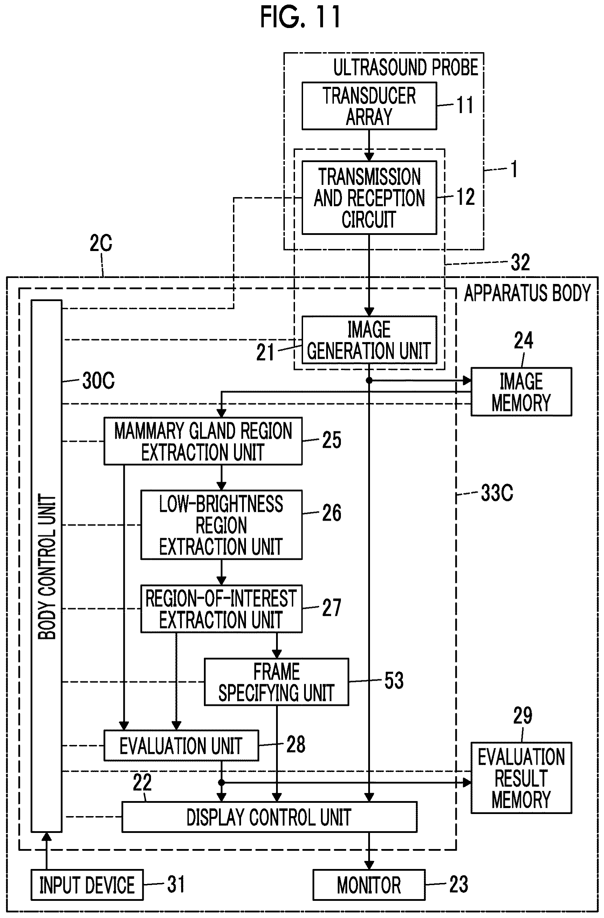

FIG. 11 shows a configuration of an ultrasound diagnostic apparatus according to Embodiment 4. The ultrasound diagnostic apparatus according to Embodiment 4 comprises an apparatus body 2C instead of the apparatus body 2 in the ultrasound diagnostic apparatus according to Embodiment 1 shown in FIG. 1. The apparatus body 2C further comprises a frame specifying unit 53 and comprises a body control unit 30C instead of the body control unit 30, in the apparatus body 2 according to Embodiment 1.

In the apparatus body 2C, the frame specifying unit 53 is connected to the region-of-interest extraction unit 27. The frame specifying unit 53 is connected to the display control unit 22 and the body control unit 30C. In addition, the image generation unit 21, the display control unit 22, the mammary gland region extraction unit 25, the low-brightness region extraction unit 26, the region-of-interest extraction unit 27, the evaluation unit 28, the body control unit 30C, and the frame specifying unit 53 constitute a processor 33C for the apparatus body 2C.

The frame specifying unit 53 specifies the frame in which the frequency is greater than the predetermined frequency threshold value, that is, the frame in which the region of interest is not extracted by the region-of-interest extraction unit 27, by performing inverse Fourier transform on the time-series data of the total area of the low-brightness region R1 in each of the ultrasound images U of the plurality of frames, the time-series data having been Fourier-transformed by the region-of-interest extraction unit 27. In this case, the frame specifying unit 53 specifies the frame in which the frequency higher than the predetermined frequency threshold value by, for example, performing inverse Fourier transform on a portion of a frequency band higher than the frequency threshold value including the portion A2 representing the presence of the fat region in the Fourier transform data shown in FIG. 7.

The display control unit 22 displays the ultrasound images U of the plurality of frames in which the region of interest has not been extracted by the region-of-interest extraction unit 27, which are specified by the frame specifying unit 53, on the monitor 23.

The user can determine the ultrasound image U of the frame that is inappropriate for the evaluation of the risk of breast cancer since the mammary gland region M includes the fat region, among the ultrasound images U of the plurality of frames in which the region of interest is not extracted by the region-of-interest extraction unit 27, which are displayed on the monitor 23, and can select the ultrasound image U of the frame.

The region-of-interest extraction unit 27 includes, in the region of interest, the low-brightness region R1 in the ultrasound image U of the frame that is not selected by the user among the ultrasound images U of the plurality of frames displayed on the monitor 23. Here, the low-brightness region R1 newly included in the region of interest is the low-brightness region R1 determined by the user as being usable for evaluating the risk of breast cancer because the fat region is effectively excluded. As described above, it is possible to improve the accuracy of the region of interest by incorporating the low-brightness region R1 in the frame that is not selected by the user into the region of interest that is automatically extracted by the region-of-interest extraction unit 27.

The evaluation unit 28 evaluates the risk of breast cancer using the region of interest output by the region-of-interest extraction unit 27 as described above.

As described above, with the ultrasound diagnostic apparatus according to Embodiment 4, the frame specifying unit 53 performs the inverse Fourier transform on the time-series data of the total area of the low-brightness region R1 in each of the plurality of frames, the time-series data having been Fourier-transformed by the region-of-interest extraction unit 27, to specify the frame in which the frequency is greater than the predetermined frequency threshold value, the display control unit 22 displays the ultrasound images U of the plurality of frames specified by the frame specifying unit 53 on the monitor 23, and the region-of-interest extraction unit 27 includes, in the region of interest, the low-brightness region R1 in the frame that is not selected by the user among the ultrasound images U of the plurality of frames displayed on the monitor 23, so that it is possible to improve the accuracy of the region of interest and to improve the accuracy of the evaluation of the risk of breast cancer by the evaluation unit 28.

The ultrasound diagnostic apparatus according to Embodiment 4 is configured by providing the apparatus body 2 with the frame specifying unit 53 in the ultrasound diagnostic apparatus according to Embodiment 1, but may be configured by providing the apparatus body 2A with the frame specifying unit 53 in the ultrasound diagnostic apparatus according to Embodiment 2, or may be configured by providing the apparatus body 2B with the frame specifying unit 53 in the ultrasound diagnostic apparatus according to Embodiment 3.

### Explanation of References

1: ultrasound probe
2: apparatus body
11: transducer array
12: transmission and reception circuit
13: pulser
14: amplifying unit
15: AD conversion unit
16: beam former
21: image generation unit
22: display control unit
23: monitor
24: image memory
25: mammary gland region extraction unit
26: low-brightness region extraction unit
27: region-of-interest extraction unit
28: evaluation unit
29: evaluation result memory
30, 30A, 30B, 30C: body control unit
31: input device
32: image acquisition unit
33, 33A, 33C: processor
41: signal processing unit
42: DSC
43: image processing unit
51: low-brightness region integration unit
52: frame interval adjustment unit
53: frame specifying unit
A1, A2, A3: portion
BR: breast region
L1: front boundary line
L2: rear boundary line
M: mammary gland region
R1: low-brightness region
R2: edematous stroma
S: skin
T: pectoralis major
U: ultrasound image

## Claims

1. An ultrasound diagnostic apparatus comprising:
an ultrasound probe;
an image acquisition unit that performs a scan with the ultrasound probe to continuously acquire ultrasound images of a plurality of frames in which a breast of a subject is imaged;
a mammary gland region extraction unit that extracts a mammary gland region from each of the ultrasound images of the plurality of frames;
a low-brightness region extraction unit that extracts a low-brightness region in which brightness is equal to or less than a predetermined brightness threshold value from the mammary gland region extracted by the mammary gland region extraction unit and that calculates an area of the low-brightness region for each frame;
a region-of-interest extraction unit that extracts a region of interest in which an area change rate is equal to or less than a predetermined change rate threshold value from a time-series change in the area of the low-brightness region in the plurality of frames; and
an evaluation unit that evaluates a risk of breast cancer based on a ratio of an area of the region of interest in the plurality of frames extracted by the region-of-interest extraction unit to an area of the mammary gland region in the plurality of frames extracted by the mammary gland region extraction unit.

2. The ultrasound diagnostic apparatus according to claim 1,
wherein the region-of-interest extraction unit selects a frame in which a frequency is equal to or less than a predetermined frequency threshold value by performing Fourier transform on time-series data of a total area of the low-brightness region in each of the plurality of frames, and extracts the low-brightness region in the selected frame as the region of interest.

3. The ultrasound diagnostic apparatus according to claim 1,
wherein the region-of-interest extraction unit calculates an area change over a predetermined number of frames for each low-brightness region in the plurality of frames, and extracts, as the region of interest, the low-brightness region in which the calculated area change is equal to or less than a predetermined area change threshold value.

4. The ultrasound diagnostic apparatus according to claim 1,
wherein the low-brightness region extraction unit extracts a pixel at which brightness is equal to or less than the predetermined brightness threshold value from the mammary gland region extracted by the mammary gland region extraction unit, and calculates an area occupied by the extracted pixel as the area of the low-brightness region for each frame.

5. The ultrasound diagnostic apparatus according to claim 1,
wherein the low-brightness region extraction unit extracts the low-brightness region by binarizing the mammary gland region extracted by the mammary gland region extraction unit based on the predetermined brightness threshold value.

6. The ultrasound diagnostic apparatus according to claim 1, further comprising:
a low-brightness region integration unit that generates a continuous low-brightness region by integrating a plurality of the low-brightness regions that are extracted by the low-brightness region extraction unit in adjacent frames among the plurality of frames and that have overlapping portions,
wherein the region-of-interest extraction unit calculates a variance of the area of the low-brightness region in each frame in the continuous low-brightness region for each continuous low-brightness region, and extracts, as the region of interest, the low-brightness region in each frame in the continuous low-brightness region in which the calculated variance is equal to or less than a predetermined variance threshold value.

7. The ultrasound diagnostic apparatus according to claim 1, further comprising:
a frame interval adjustment unit that calculates a position change amount of an imaging point in the ultrasound images of adjacent frames among the plurality of frames and that adjusts a time interval of the adjacent frames in accordance with the calculated position change amount,
wherein the region-of-interest extraction unit extracts the region of interest from the time-series change in the area of the low-brightness region in the plurality of frames based on the time interval adjusted by the frame interval adjustment unit.

8. The ultrasound diagnostic apparatus according to claim 2, further comprising:
a monitor that displays the ultrasound image; and
a frame specifying unit that specifies a frame in which a frequency is greater than the predetermined frequency threshold value by performing inverse Fourier transform on the time-series data of the total area of the low-brightness region in each of the plurality of frames, the time-series data having been Fourier-transformed by the region-of-interest extraction unit,
wherein the ultrasound image of the frame specified by the frame specifying unit is displayed on the monitor.

9. A method of controlling an ultrasound diagnostic apparatus, the method comprising:
performing a scan with an ultrasound probe to continuously acquire ultrasound images of a plurality of frames in which a breast of a subject is imaged;
extracting a mammary gland region from each of the ultrasound images of the plurality of frames;
extracting a low-brightness region in which brightness is equal to or less than a predetermined brightness threshold value from the extracted mammary gland region and calculating an area of the low-brightness region for each frame;
extracting a region of interest in which an area change rate is equal to or less than a predetermined change rate threshold value from a time-series change in the area of the low-brightness region in the plurality of frames; and
evaluating a risk of breast cancer based on a ratio of an area of the extracted region of interest in the plurality of frames to an area of the extracted mammary gland region in the plurality of frames.
